# EUROPÄISCHE PATENTSCHRIFT

(11) **EP 2 388 128 B1**
(45) Veröffentlichungstag und Bekanntmachung des Hinweises auf die Patenterteilung: **06.09.2017**
(21) Anmeldenummer: 11166850.5
(22) Anmeldetag: 20.05.2011
(51) Int. Cl.: B29C 49/42, B29C 49/46, A61L 2/02

(54) **Vorrichtung und Verfahren zur Reinigung von Gas in Blasmaschinen**
Method and device for cleaning gas in blowing machines
Dispositif et procédé de nettoyage de gaz dans des souffleuses

(30) Priorität: 20.05.2010 DE 102010022129
(43) Veröffentlichungstag der Anmeldung: 23.11.2011
(73) Patentinhaber: Krones AG, 93073 Neutraubling (DE)
(72) Erfinder: Dahmen, Michael, 93073 Neutraubling (DE); Lappe, Ulrich, 93073 Neutraubling (DE); Handschuh, Eduard, 93073 Neutraubling (DE); Martini, Oliver, 93073 Neutraubling (DE); Söllner, Jürgen, 93073 Neutraubling (DE); Neubauer, Michael, 93073 Neutraubling (DE)
(74) Vertreter: Bittner, Bernhard

(56) Entgegenhaltungen:
- DE-A1- 19 919 623
- DE-T2- 69 520 445
- US-A- 8 180
- US-A- 3 650 678
- US-A- 4 714 647
- US-A- 5 759 218
- US-A1- 2002 159 915
- US-A1- 2006 185 321
- US-B1- 7 393 373
- US-B2- 7 128 872
- US-B2- 7 157 046

## Beschreibung

Die Erfindung betrifft eine Vorrichtung und ein Verfahren zur Reinigung von Gas in Blasmaschinen zum Umformen von Kunststoffvorformlingen zu Kunststoffbehältnissen.

Blasmaschinen zum Umformen von Kunststoffvorformlingen zu Kunststoffbehältnissen sind aus dem Stand der Technik seit langem bekannt. Üblicherweise weisen solche Vorrichtungen eine Vielzahl von Blasformen auf, welche jeweils einen Hohlraum ausbilden, innerhalb dessen die Kunststoffvorformlinge zu Behältnissen umformbar sind innerhalb derer Vorformlinge aus Kunststoff (Kunststoffvorformlinge) zu Kunststoffbehältnissen expandiert werden. Zur Expansion der Kunststoffvorformlinge weist die Vorrichtung eine Druckbeaufschlagungseinrichtung auf, die die Kunststoffvorformlinge mit einem Medium, wie beispielsweise Druckluft, beaufschlagt. Dadurch wird der Kunststoffvorformling sowohl radial als auch in Längsrichtung gedehnt und gegen eine den Hohlraum begrenzende Innenwand der Blasform gedrückt. Zur Unterstützung der Dehnung in Längsrichtung des Kunststoffbehältnisses weisen Blasmaschinen üblicherweise eine Reckstange auf. Stromabwärts bezüglich dieser Blaseinrichtungen befinden sich üblicherweise weitere Anlagen zum Behandeln der Behältnisse wie beispielsweise Desinfektionseinrichtungen, Befüllungsanlagen, Verschließer und dergleichen.

Zu einer besseren Nutzung des Gas-Überdruckes, mit dem die Kunststoffvorformlinge in den Blasformen zu Kunststoffbehältnissen expandiert werden, ist in einigen Blasmaschinen vorgesehen, den noch in dem geformten Kunststoffbehältnis vorhandenen Überdruck nicht unkontrolliert entweichen zu lassen, sondern zurückzuführen und das Gas wieder zu verwenden. Dies kann beispielsweise dann besonders vorteilhaft sein, wenn in einer Blasmaschine die Kunststoffvorformlinge zunächst mit einem geringeren Gasdruck beaufschlagt werden, um (evtl. parallel zur Streckung des Kunststoffvorformlings mittels einer Reckstange) eine Vordehnung auszuführen. Dieser geringere Gasdruck kann beispielsweise der Abgasdruck aus dem finalen Streckprozess anderer Kunststoffbehältnisse sein. Damit ist es möglich, den in den gestreckten Gebinden verbleibenden Überdruck zu nutzen und damit die Effizienz der Blasmaschine zu erhöhen. Durch eine derartige maschineninterne Blasluftrückgewinnung kann erreicht werden, dass von außen keine Reck- oder Vorblasluft mehr zugeführt werden muss.
Besonders bei modernen Blasmaschinen mit sehr hohen Durchsatzleistungen ist eine Nutzung des in den Kunststoffbehältnissen verbleibenden Gasdruckes sinnvoll und ökonomisch vorteilhaft. Durch die Möglichkeit, die Pumpenleistung zur Erzeugung des Überdruckes zu reduzieren, können die Betriebskosten gesenkt und Emissionen reduziert werden. So ist es möglich, die durch derartige Recyclingprozesse zurück gewonnene Luft in einem Ringkanal zu sammeln und wiederum zum Blasen der nachfolgenden Behältnisse zu verwenden.
Die Rückführung der Medien zur Druckbeaufschlagung der Kunststoffvorformlinge birgt jedoch die Gefahr, dass Verunreinigungen z.B. aus den Vorformlingen mit dem Medium verschleppt werden und somit eine Vielzahl von Blasformen kontaminiert werden. Dies ist insbesondere bei Blasmaschinen mit erhöhtem Hygienelevel kritisch und hier besonders hinsichtlich mikrobiologischer Verunreinigungen gefährlich, da so eventuell schädigende Mikroorgansimen bis hin zu Krankheitserregern in eine große Anzahl von Kunststoffbehältnissen gelangen können und somit auch in das Produkt, welches in die ausgeformten Kunststoffbehältnisse gefüllt wird. US-A-3650678 offenbart den Oberbegriff der Ansprüche 1, 12. Die Aufgabe der vorliegenden Erfindung besteht demnach darin, eine Gasaufbereitungseinrichtung bereit zu stellen, die es ermöglicht, die in einer Blasmaschine - insbesondere zwischen einem Reservoir und einer Blasform verlaufenden - Gasströme zu reinigen. Insbesondere sollen mikrobiologische Verunreinigungen sowie Reste von Desinfektions-/Sterilisationsmitteln beseitigt oder zumindest deren Anteil drastisch reduziert werden. Des Weiteren ist es Aufgabe der vorliegenden Erfindung, ein Verfahren zur Reinigung der zwischen einem Reservoir und mindestens einer Blasform in einer Blasmaschine verlaufenden Gasströme bereit zu stellen.

Diese Aufgabe wird erfindungsgemäß durch eine Vorrichtung gemäß Anspruch 1 gelöst. Es wird darauf hingewiesen, dass im folgenden die Begriffe Desinfektionsmittel und Sterilisationsmittel auch synonym verwendet werden können.
Durch eine derartige Vorrichtung ist es möglich, Verunreinigungen in dem Druckgasversorgungssystem zu reduzieren und mikrobielle Verunreinigungen evtl. vollständig zu vermeiden. Zumindest wird bei einer Rückleitung des verwendeten Gases und dessen Wiederverwendung die Verteilung von Verunreinigungen auf eine Vielzahl von in der Blasmaschine umgeformten Kunststoffbehältnissen vermieden.
Aufgrund der Eignung und der einfachen Verfügbarkeit ist besonders Luft als gasförmiges Medium zur Verwendung in Blasmaschinen geeignet. In speziellen Fällen können jedoch auch andere Gase vorteilhaft sein und zum Einsatz kommen. So können beispielsweise sterile Gase wie Sterilluft, nicht brandfördernde Gase wie Stickstoff oder je nach Anwendungsbereich auch andere gasförmigen, wie beispielsweise sterilisierende Medien zum Expandieren der Behältnisse verwendet werden.

Dabei ist die Gasaufbereitungseinrichtung in Form eines Sterilfilters in einer Druckgasleitung angeordnet, die zu einer Blasform führt und kann in Form eines Sterilfilters in einer Druckgasleitung, die von einer Blasform abführt, angeordnet werden. Besonders vorteilhaft ist die Anordnung in einer Druckgasableitung, wenn diese zu einem Druckgasreservoir führt und somit eine Wiederverwertung des Gases erfolgen kann. Ein Sterilfilter in der zu einer Blasform führenden Druckgasleitung filtert das der Blasform zugeführte Druckgas steril und verhindert das Einbringen von evtl. in dem Druckgas oder im Druckgasreservoir vorhandenen Mikroorganismen in das herzustellende Kunststoffbehältnis. Da jedoch auch die in die Blasmaschine eingebrachten Kunststoffvorformlinge verunreinigt und mikrobiell kontaminiert sein können, ist es sinnvoll, auch in der von der Blasform zu einem Druckgasreservoir führende Leitung mit einem Sterilfilter zu versehen, um das Einschleppen von Verunreinigungen (durch den Kunststoffvorformling) beim Entspannen in das Druckgasreservoir zu vermeiden. Hierbei sind neben mikrobiellen Verunreinigungen auch beispielsweise Staub und Feinstaub von besonderem Interesse, da sie durch die während des Blasprozesses in dem Kunststoffbehältnis entstehenden Gasbewegungen aufgewirbelt werden können und somit in den Druckgasstrom gelangen können. Derartige Verunreinigungen können durch einen entsprechenden Filter ebenfalls abgetrennt werden, wodurch eine Anreicherung im Druckgasreservoir vermieden wird.
Um mikrobielle Verunreinigungen der Kunststoffbehältnisse zu vermeiden, ist es möglich, diese durch geeignete Verfahren zu desinfizieren oder zu sterilisieren. Als Desinfektions/- Sterilisationsmittel werden häufig oxidative Desinfektions-/Sterilisationsmittel eingesetzt. Insbesondere Peroxide, wobei besonders Wasserstoffperoxid (H₂O₂) aufgrund seiner hohen mikrobioziden Wirkung, der Umweltverträglichkeit und der einfachen technischen Umsetzbarkeit besonders oft verwendet wird. Jedoch ist es nach Möglichkeit zu vermeiden, Peroxide unkontrolliert anzureichern, da sie in der Regel brandfördernd sind und im Extremfall sogar explosiv sein können. Besonders in Anwesenheit von Metallionen neigt auch Wasserstoffperoxid zur Zersetzung, bei der große Mengen Energie freigesetzt werden. Daher ist es wichtig, das Druckgas von Resten dieser oxidativen Desinfektions-/Sterilisationsmittel zu befreien. Dadurch wird auch eine Verschleppung der oxidativen Desinfektions-/Sterilisationsmittel in umzuformende Kunststoffvorformlinge und eine mögliche Anreicherung in diesen Eine weitere mögliche Ausführung einer Gasaufbereitungseinrichtung kann einen Katalysator umfassen, der optional auch zusätzlich zu einer Filtereinrichtung verwendet werden kann. Ein derartiger Katalysator reduziert die Menge an oxidativen Desinfektions-/Sterilisationsmitteln in dem Druckgas. Wasserstoffperoxid kann in einem derartigen Katalysator beispielsweise zu Wasser und Sauerstoff zersetzt werden. Durch eine einem Filter vorgeschaltete Druckgasaufbereitung mittels eines Katalysators kann bereits vor dem Durchleiten durch den Filter der Anteil oxidativer Reagenzien stark reduziert werden, so dass die Lebensdauer des Filters verlängert werden kann. Daher ist abhängig vom Anwendungsbereich auch eine Kombination aus Sterilfilter und Katalysator vorteilhaft. Erfindungsgemäß ist die mindestens eine Gasaufbereitungseinrichtung ein Filter, welcher in der Lage ist Gas steril zu filtern und dieser Filter ist in einer Gasleitung angeordnet, durch welche Druckgas zu einer Blasstation zuführbar ist.
Durch eine derartige Ausführungsform ist es möglich, das Druckgas zu filtern, das der jeweiligen Blasstation zugeführt wird. Eventuell bereits in dem Druckgas enthaltene Kontaminationen können so zurückgehalten werden. Somit wird auch das Einbringen von Verunreinigungen in das in der jeweiligen Blasform vom Kunststoffvorformling zum Kunststoffbehältnis umgeformte Gebinde vermieden. Damit ist es möglich, die Intervallzeiten zwischen einzelnen Desinfektionszyklen der einzelnen Blasformen zu verlängern, wodurch sich eine höhere Gesamtleistung der Blasmaschine ergibt.

In einer weiteren bevorzugten Ausführungsform der Vorrichtung umfasst die mindestens eine Gasaufbereitungseinrichtung einen Filter, welcher in der Lage ist Gas steril zu filtern und dieser Filter ist in einer Gasleitung angeordnet, durch welche Gas von einer Blasstation abführbar ist. Eine derartige Ausführungsform dient vornehmlich der Vermeidung von Kontaminationen des Druckgasreservoirs. Im Fall der Rückgewinnung des Druckgases besteht ohne einen derartigen Sterilfilter die Gefahr, dass Verunreinigungen wie beispielsweise Mikroorgansimen, welche sich möglicherweise in einem einzelnen der der Blasmaschine zugeführten Kunststoffvorformlinge befinden, über das Druckgasrückgewinnungssystem in eine Vielzahl von Kunststoffbehältnissen eingeschleppt werden. Daher ist es vorteilhaft, bereits das Druckgas steril zu filtern, welches von den Blasformen dem Druckgasreservoir zugeführt wird. Einer Anreicherung und möglicherweise Vermehrung der mikrobiellen Verunreinigung im Druckgasreservoir wird somit vorgebeugt.

In einer weiteren bevorzugten Ausführungsform der Vorrichtung umfasst die mindestens eine Gasaufbereitungseinrichtung einen Katalysator, welcher dazu geeignet ist, in durchströmenden und/oder vorbeiströmenden Gas enthaltene oxidative Desinfektions-/Sterilisationsmittel in ihrer Menge zu reduzieren und dieser Katalysator ist in einer Gasleitung angeordnet, durch welche Gas von einer Blasstation abführbar ist. Durch die Anordnung eines Katalysators in der Gasleitung mittels welcher Gas aus der jeweiligen Blasform abgeführt wird, kann die Menge von oxidativen Desinfektions-/Sterilisationsmitteln drastisch reduziert werden. Desinfektions-/Sterilisationsmittel können sowohl über verbleibende Reste in eventuell sterilisierten Kunststoffvorformlingen eingebracht werden oder die Folge einer Sterilisation der jeweiligen Blasformen sein. Aufgrund des hohen Oxidationspotentials und der Zersetzungsenergie ist es erforderlich, derartige oxidative Desinfektions-/Sterilisationsmittel unschädlich zu machen, um eine unkontrollierte Anreicherung zu vermeiden. Eine derartige Anreicherung kann im Extremfall zu einer Explosion führen. Aber auch ohne eine Explosion bergen die üblicherweise verwendeten oxidativen Desinfektions-/Sterilisationsmittel die Gefahr ihrer brandfördernden Eigenschaft. Daher ist eine unkontrollierte Anreicherung unbedingt zu vermeiden.

Da ein besonders häufig verwendetes oxidatives Desinfektions-/Sterilisationsmittel Wasserstoffperoxid (H₂O₂) ist, ist in einer bevorzugten Ausführungsform der Vorrichtung der mindestens eine Katalysator ein Katalysator, welcher dazu geeignet ist, die Menge an H₂O₂ in der Gas zu reduzieren. Die hohe bakterizide Wirkung von H₂O₂, dessen Umweltverträglichkeit und die einfache Möglichkeit der technischen Umsetzung einer Desinfektion/Sterilisation mit H₂O₂ machen es zu einem bevorzugten oxidativen Desinfektions-/Sterilisationsmittel. Durch etliche verschiedene Katalysatoren kann H₂O₂ zu Wasser (H₂O) und Sauerstoff (O₂) umgesetzt und damit unschädlich gemacht werden. Es wäre auch möglich, mehrere Katalysatoren, welche in Reihe geschaltet sind, zu verwenden, um auf diese Weise mehrere oxidative Komponenten aus dem Luftstrom zu entfernen bzw. zu reduzieren.

Zur Umsetzung von oxidativen Desinfektions-/Sterilisationsmitteln sind verschiedene Katalysatoren geeignet. In einer besonderen Ausführungsform der Vorrichtung umfasst der mindestens eine Katalysator ein Metall, welches bevorzugt aus einer Gruppe ausgewählt ist, die Platin (z.B. in Form eines Platingewebes), Palladium, Nickel, Gold, Silber, Kupfer, Rhodium, Kobalt, Osmium, Eisen, Chrom, Vanadium, Zirkonium, Hafnium, Cer, Samarium, Zink, Mangan und Kombinationen hiervon und dergleichen umfasst. Neben den besonders oft verwendeten Katalysatoren, die Platin oder Metalle der Platingruppe enthalten, bieten sich manganenthaltende Katalysatoren an. So ist beispielsweise Braunstein (MnO₂) als effizienter und Kostengünstiger Katalysator für die Spaltung von H₂O₂ zu Wasser (H₂O und Sauerstoff (O₂) bekannt.

Bei einer weiteren vorteilhaften Ausführungsform weist die Vorrichtung einen Schalldämpfer insbesondere für austretende Prozessluft auf und dieser Schalldämpfer weist bevorzugt einen Katalysator auf. Bevorzugt ist daher in einer weiteren bevorzugten Ausführungsform mindestens einer der Katalysatoren mit einem Schalldämpfer kombiniert und/oder in einen Schalldämpfer integriert. Eine derartige Kombination von Katalysator und Schalldämpfer ist besonders günstig, da beide Elemente eine möglichst große innere Oberfläche aufweisen sollten, um einerseits eine große Kontaktfläche mit dem Katalysatormaterial für die zu spaltenden Desinfektions-/Sterilisationsmittel bereitzustellen und andererseits viele Reflektionsflächen für den Schall zu bieten. Bevorzugt wird das Katalysatormaterial als (evtl. dünne) Schicht auf ein poröses Matrixmaterial aufgebracht. Durch die Porosität wird sowohl eine große Oberfläche für die Reaktion mit den Resten der Desinfektions-/Sterilisationsmittel erzeugt als auch der Schall gedämpft. Eine Reihenschaltung von Katalysator und Schalldämpfer, bei der Katalysator und Schalldämpfer als separate Elemente ausgebildet sind ist daher nicht notwendig. Dies führt zu einer möglichen kompakteren Ausführung.

In einer Ausführungsform kann das Innere eines Schalldämpfers mit Katalysatormaterial beschichtet werden oder Katalysatormaterial zusätzlich in den Schalldämpfer eingebracht werden. Die schalldämpfenden Eigenschaften können durch eine spezielle Formgebung des Schalldämpferinnenraums verstärkt werden. Besonders bevorzugt sind verwinkelte, besonders bevorzugt unregelmäßige Oberflächen. Es ist jedoch auch möglich, dass das Katalysatormaterial bereits schalldämmende Eigenschaften aufweist und/oder als schalldämmendes Material wirkt. Dies kann beispielsweise dadurch erreicht werden, dass der Katalysator in Form eines Granulats, Schwamms, Netzes oder anderer Formen mit großer spezifischer Oberfläche verwendet wird. So können Schallwellen beispielsweise in die Poren des Katalysatorschwamms oder in die Zwischenräume zwischen Granulatpartikeln eindringen wo sie zwischen den jeweiligen Oberflächen ständig reflektiert werden, bis die Intensität stark nachlässt und die Schallwelle ausläuft, bzw. stark geschwächt ist. In diesem Fall bestehen ganze Bereiche des Schalldämpfers, wie beispielsweise die Wände oder ein Füllmaterial aus dem Katalysatormaterial.

Es ist auch möglich, dass das Schalldämpfermaterial bereits eine sehr große innere Oberfläche aufweist und lediglich, z.B. nachträglich, mit dem Katalysatormaterial beschichtet wird. Dies kann beispielsweise durch Aufdampfen, elektrolytische Abscheidung oder eine andere Art der Abscheidung erfolgen. Es ist demnach sowohl möglich, das Katalysatormaterial direkt in den Schalldämpfer einzubringen (z.B. als Granulat) bzw. die schalldämpfenden Eigenschaften direkt durch das Katalysatormaterial zu erreichen, als auch das Katalysatormaterial als katalytisch aktive Schicht auf das schalldämpfende Material aufzubringen.

Filter zum Sterilfiltern sind allgemein bekannt und mit verschiedenen Porengrößen kommerziell erhältlich. Zum Filtrieren von mikrobiellen Verunreinigungen aus Gasen sind jedoch trotz großer Durchflussleistung besonders geringe Porengrößen möglich. In einer besonders bevorzugten Ausführungsform der Vorrichtung weist daher der mindestens eine Filter zum Sterilfiltern von Gas Porengrößen von weniger als 50 µm, bevorzugt von weniger als 5 µm, besonders bevorzugt von weniger als 0,5 µm auf. Durch diese geringen Porengrößen ist es möglich mikrobielle Verunreinigungen effizient aus der durch den Filter strömenden Gas herauszufiltern. Gleichzeitig ist jedoch weiterhin ein ausreichend großes Durchflussvolumen durch den Filter möglich.

Diese Sterilfilter und insbesondere die Filtermembran bestehen bevorzugt aus einem Material, welches zu dem insbesondere für die Desinfektion / Sterilisation der Kunststoffvorformlinge bzw. Kunststoffbehältnisse verwendeten Desinfektions-/Sterilisationsmittel kompatibel ist und bevorzugt durch dieses nicht angegriffen wird. Im Sterilfilter werden demnach nur Werkstoffe eingesetzt, welche gegenüber dem Desinfektions-/Sterilisationsmittel beständig sind. Eine unerwünschte chemische Reaktion des Desinfektions-/Sterilisationsmittels mit dem Sterilfilter und insbesondere der Filtermembran ist somit nicht möglich. Dies ermöglicht die Desinfektion/Sterilisation der Sterilfilter mit dem jeweiligen Desinfektions-/Sterilisationsmittel, was die Standzeiten der Anlage deutlich reduzieren kann. Die Filter müssen für deren Reinigung nicht ausgebaut werden, sondern können gemeinsam mit dem Leitungssystem desinfiziert werden. Weiterhin ermöglicht dies den Einsatz dieser Sterilfilter an vielen verschiedenen Positionen innerhalb des Leitungssystems für die beim (Streck-) Blasprozess verwendeten Gase. So kann beispielsweise ein zentraler Sterilfilter in der Zuführleitung vor einem Ringkanal angeordnet sein. Dies erfordert Filter, die für einen großen Volumendurchsatz ausgelegt sind. Das gesamte für die (Streck-) Blasprozesse in den möglicherweise sehr vielen (Streck-) Blaseinrichtungen benötigte Druckgas passiert diesen Filter und wird von diesem gereinigt.

In einer weiteren Ausführungsform ist vorgesehen, jeweils mindestens einen der gegenüber dem Desinfektions- bzw. Sterilisationsmittel inerten Filter in eine Leitung zu integrieren, welche direkt zu einer der vielen (Streck-) Blaseinrichtungen führt. Bevorzugt führen diese Leitungen von einem Verteilsystem wie z.B. einem Ringkanal zu der jeweiligen (Streck-) Blaseinrichtung. Durch diese Anordnung wird die durch jeden einzelnen Filter geleitete Gasmenge deutlich reduziert und die Reinigungs- bzw. Desinfektionsintervalle können verlängert werden. Weiterhin ist es bei dieser Ausführungsform möglich, die jeweiligen Sterilfilter kleiner auszuführen, wodurch eine Kostenreduzierung erreicht werden kann. Im Fall eines Defekts einer der Filter kann dieser mit einem vergleichsweise geringen Kostenaufwand ausgetauscht werden.

Um die Filter bei vorgegebener Reinigungsleistung möglichst kompakt ausführen zu können ist eine optimale Verteilung der Strömungsverhältnisse über die Filterfläche vorteilhaft. Insbesondere bei der Anordnung in der direkten Gaszuleitung zu einer (Streck-) Blaseinrichtung ist das Raumangebot beschränkt, so dass kompakte Bauweisen für die Filter bevorzugt sind. Um in diesem Fall eine ausreichende Reinigungsleistung zu erreichen wird vorgeschlagen, die Gaszu- und ableitungen am Sterilfilter nicht symmetrisch zur Längsachse des Sterilfilters anzuordnen, sondern versetzt gegenüber dieser. Die Zu- und Abluftanschlüsse sind demnach außermittig gesetzt, was zu einer Vergrößerung der luftdurchsetzten Fläche (insbesondere der Membranfläche) führt. Dadurch wird vermieden, dass laminare, aus der Zuleitung in den Sterilfilter einströmendes Gas die Filterfläche im Zentrum mehr verschmutzt als in den Randbereichen. Insbesondere bei Ausführungen mit mehreren Filtermembranen, wobei diese jeweils ebenfalls gegenüber einander und gegenüber der zentralen Achse des Sterilfilters versetzt angeordnet sind, kann ein turbulenter Strömungsverlauf des Gasstroms erzwungen werden, wodurch eine weitgehend gleichmäßige Beaufschlagung der einzelnen Membranen mit Gas erfolgt. Durch diese Optimierung der Luftverteilung im Sterilfilter kann das Filtermaterial besser ausgenutzt werden, was eine kompaktere Bauweise des Sterilfilters ermöglicht.

Die Zuführung des Desinfektions-/Sterilisationsmittels zu dem Sterilfilter kann durch die Druckmediumleitung oder durch eine separate Leitung erfolgen.

Zur Überwachung der Reinigungsleistung ist in einer bevorzugten Ausführungsform der Vorrichtung in mindestens einer der Gasleitungen stromabwärts bezüglich mindestens eines Filters oder eines Katalysators eine Messvorrichtung angeordnet, welche dazu geeignet ist, die Reinigungsleistung des stromaufwärts angeordneten Filters oder Katalysators zu messen. Dadurch kann die Qualität des Druckgases ständig kontrolliert werden und abhängig von diesen Messwerten die Intervallzeit für notwendige Reinigungszyklen des stromaufwärts angeordneten Filters oder Katalysators oder auch der gesamten Blasmaschine ermittelt werden. Die Anzahl der Reinigungs- oder Desinfektionszyklen kann somit reduziert und die Effizienz der Blasmaschine erhöht werden. Weiterhin ist es möglich, durch die Anordnung von mehreren Messvorichtungen den Ort der Verunreinigung zu ermitteln und somit die Wartungsarbeiten auf spezielle Segmente der Blasmaschine zu beschränken. Eventuell ist bereits der Austausch einer einzelnen Gasaufbereitungseinrichtung ausreichend, um eine ausreichende Qualität des Druckgases und der Gasleitungssysteme wiederherzustellen.

Es wäre jedoch auch möglich, eine zentrale Messvorrichtung beispielsweise an einem Rückluftauffangbehälter anzuordnen. Auch könnte die Messvorrichtung direkt in den Katalysator integriert bzw. eingebaut sein.

Für den Fall, dass eine der Messvorrichtungen ein Messergebnis liefert, dass auf eine Kontamination des jeweilligen Bereiches hinweist, ist es zweckmäßig, diesen Bereich schnellstmöglich von anderen noch nicht kontaminierten Bereichen abzutrennen, um eine weitere Ausbreitung der Verunreinigung zu vermeiden. In einer besonders bevorzugten Ausführungsform der Vorrichtung ist daher in mindestens einer der Gasleitungen stromabwärts bezüglich mindestens einer Messvorrichtung eine Absperrvorrichtung zum Verschließen der Gasleitung angeordnet. Eine geeignete Absperrvorrichtung zum Verschließen der Gasleitung kann beispielsweise ein Ventil sein, welches die kontaminierte Leitung verschließen kann.

Um das Absperren einer Gasleitung abhängig von dem Signal der Messvorrichtung auszulösen ist in einer besonders bevorzugten Ausführungsform der Vorrichtung vorgesehen, dass die Absperrvorrichtung zum Verschließen der Gasleitung mit mindestens einer Steuereinrichtung verbunden ist, welche dazu geeignet ist, die Absperrvorrichtung abhängig von den von der Messvorrichtung ermittelten Werten zu verschließen oder zu öffnen. Die Steuereinrichtung ist in der Lage, die von der Messvorrichtung ermittelten Werte auszuwerten und daraus entsprechende Rückschlüsse auf die Gasqualität und damit auch auf die Verunreinigungen abzuleiten. Als Resultat dieser Auswertung werden Steuersignale an die Absperrvorrichtung gesandt, welche diese dazu veranlassen, die entsprechende Gasleitung zu verschließen. Ebenso kann durch die Steuereinheit auch nach einer evtl. erfolgten Beseitigung der Verunreinigung in dem betreffenden Leitungsabschnitt auch die Öffnung der Absperrvorrichtung erfolgen. Ein manuelles Eingreifen zum Schließen der Absperrvorrichtung ist in dieser automatisierten Ausführungsform nicht notwendig. Dadurch kann auf eine Kontamination in einem Leitungsabschnitt sehr schnell automatisch reagiert werden und eine Kontamination weiterer Bereiche der Blasmaschine vermieden werden. Auch wäre es möglich, falls die Sterilisation des Leitungsnetzes mittels eines oxidativen Agens vorgenommen wird, den Katalysator oder andere Filtereinrichtungen durch die Absperrvorrichtung entsprechend zu schützen. Durch das Vorsehen von Katalysatoren ist es möglich, oxidative Komponenten, welche möglicherweise negative Einflüsse auf die Materialbeständigkeit haben könnten, oder eine Wiederverwertung der Druckluft einschränken, entfernt bzw. unschädlich gemacht werden. Ebenso kann verhindert werden, dass sich die oxidative Komponente in der rückgewonnenen und wieder eingesetzten Blasluft anreichert und sich somit höhere Restkonzentrationen am oxidativen Agens in der frisch geblasenen Flasche ergeben. Durch den Einbau des Katalysators können somit auch bei vorangegangener Entkeimung des Vorformlings Systeme zur Blasluftrückgewinnung eingesetzt werden.

Besonders vorteilhaft ist eine erfindungsgemäße Vorrichtung, wenn die Blasmaschine in einem Reinraum angeordnet ist, oder sie einen Reinraum aufweist, innerhalb dessen die Behältnisse transportiert werden. Dabei kann beispielsweise eine Vorrichtung vorgesehen sein, wie sie in der WO 2010/020 529 A2 beschrieben wurde. Der Gegenstand dieser Offenbarung wird hiermit durch Bezugnahme voll umfänglich zum Gegenstand auch der vorliegenden Offenbarung gemacht.

Ein weiterer wesentlicher Aspekt der Erfindung zur Lösung der gestellten Aufgabe ist ein Verfahren zur Reinigung von Gas gemäß Anspruch 12. Durch dieses Verfahren ist es möglich, Gas im Inneren einer Blasmaschine zu reinigen. Durch das Durchleiten des in Blasmaschinen zum Umformen von Kunststoffvorformlingen zu Kunststoffbehältnissen benötigten Druckgases durch entsprechende Gasaufbereitungseinrichtungen kann die Verunreinigung und/oder Kontamination sowohl der fertigen Kunststoffbehältnisse als auch des (Druck-)-Gasversorgungssystems vermieden oder zumindest stark reduziert werden.

Weitere Vorteile, Ziele und Eigenschaften vorliegender Offenbarung werden anhand nachfolgender, der Beschreibung anliegender Zeichnungen erläutert, in welchen beispielhaft eine Vorrichtung zur Reinigung von Gas in Blasmaschinen zum Umformen von Kunststoffvorformlingen zu Kunststoffbehältnissen dargestellt ist.
Es zeigen:
Fig. 1 eine schematische Darstellung einer Anlage zum Herstellen von Kunststoffbehältnissen nach dem Stand der Technik;
Fig. 2 eine Ansicht eines Reinraums im Bereich einer Blasstation nach dem Stand der Technik;
Fig. 3: eine schematische Darstellung einer Vorrichtung mit Verbindungsleitungen zwischen einem Reservoir und einer einzelnen Blasstation,
Fig. 4: eine schematische Darstellung eines Systems von Leitungen mit verschiedenen Gasdrücken innerhalb einer Blasmaschine,
Fig. 5a - 5c verschiedenen Ausführungsvarianten eines Schalldämpfers mit integriertem Katalysator,
Fig. 6a, 6b verschiedene Anordnungen des/der erfindungsgemäßen Steritfilter im Leitungssystem und
Fig. 7a, 7b verschiedene Ausführungsformen der Sterilfilter.

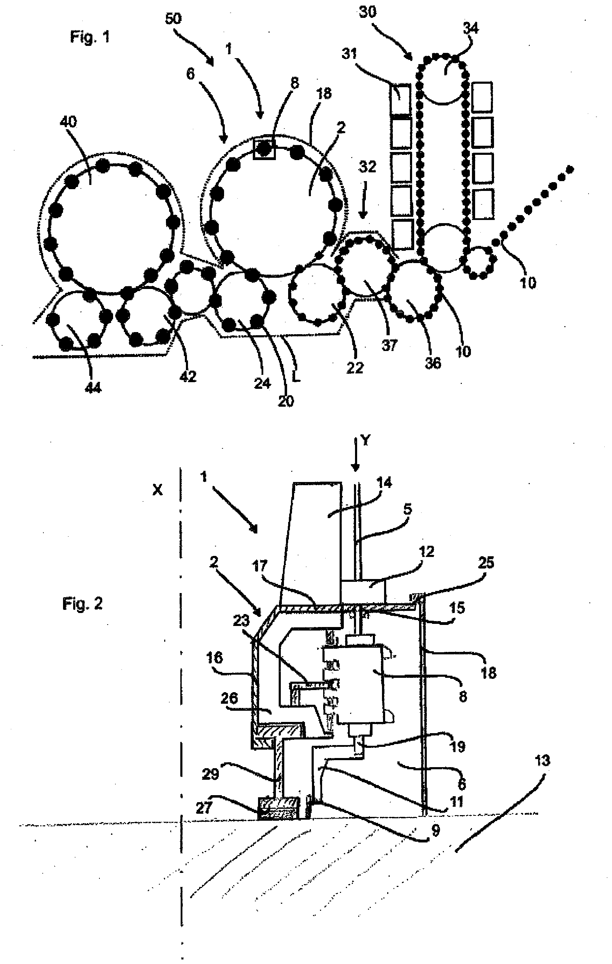

Figur 1 zeigt eine schematische Darstellung einer Anlage nach dem Stand der Technik zum Herstellen von Kunststoffbehältnissen. Diese Anlage 50 weist eine Heizeinrichtung 30 auf, in der Kunststoffvorformlinge 10 erwärmt werden. Dabei werden diese Kunststoffvorformlinge 10 mittels einer Transporteinrichtung 34, wie hier einer umlaufenden Kette, durch diese Heizeinrichtung 30 geführt und dabei mit einer Vielzahl von Heizelementen 31 erwärmt. An diese Heizeinrichtung 30 schließt sich eine Übergabeeinheit 36 an, welche die Vorformlinge 10 an eine Sterilisationseinrichtung 32 übergibt. Diese Sterilisationseinrichtung 32 weist dabei hier ebenfalls ein Transportrad 37 auf und an diesem Transportrad 37 oder auch stationär können Sterilisationselemente angeordnet sein. In diesem Bereich ist beispielsweise eine Sterilisation durch Wasserstoffperoxidgas oder auch, wie oben erwähnt, durch elektromagnetische Strahlung möglich. Insbesondere wird in diesem Bereich eine Innensterilisation der Vorformlinge durchgeführt.

Das Bezugszeichen 6 bezeichnet in seiner Gesamtheit einen Reinraum, dessen Außenbegrenzungen hier durch die punktierte Linie L angedeutet sind. Man erkennt, dass dieser Reinraum 6 in dem Bereich der Sterilisationseinheit 32 beginnt. In diesem Bereich können Schleuseneinrichtungen vorgesehen sein, um die Kunststoffvorformlinge in den Reinraum 6 einzuführen, ohne dass dabei zu viel Gas innerhalb des Reinraums verloren geht.

Der Reinraum ist, wie durch die gestrichelte Linie L angedeutet, an die Außengestalt der einzelnen Anlagenkomponenten angepasst. Auf diese Weise kann das Volumen des Reinraums reduziert werden.

Das Bezugszeichen 1 bezeichnet in ihrer Gesamtheit eine Umformungsvorrichtung, bei der an einem Transportrad 2 eine Vielzahl von Blasstationen 8 angeordnet ist, wobei hier lediglich einer dieser Blasstationen 8 dargestellt ist. Mit diesen Blasstationen 8 werden die Kunststoffvorformlinge 10 zu Behältnissen 20 expandiert. Obwohl hier nicht detailliert gezeigt, befindet sich nicht der gesamte Bereich des Transporteinrichtung 2 innerhalb des Reinraums 6, sondern der Reinraum 6 bzw. Isolator ist gewissermaßen als Mini-Isolator innerhalb der gesamten Vorrichtung realisiert. So wäre es möglich, dass der Reinraum zumindest im Bereich der Umformungsvorrichtung 1 kanalartig ausgeführt ist.

Das Bezugszeichen 22 bezieht sich auf eine Zuführeinrichtung, welche die Vorformlinge an die Umformungseinrichtung 1 übergibt und das Bezugszeichen 24 auf eine Abführeinrichtung, welche die hergestellten Kunststoffbehältnisse 20 von der Umformungsvorrichtung 1 abführt. Man erkennt, dass der Reinraum 6 in dem Bereich der Zuführeinrichtung 22 und der Abführeinrichtung 24 an jeweils Ausnehmungen aufweist, welche diese Einrichtungen 22, 24 aufnehmen. Auf diese Weise kann in besonders vorteilhafter Weise eine Übergabe der Kunststoffvorformlinge 10 an die Umformungsvorrichtung 1 bzw. eine Übernahme der Kunststoffbehältnisse 20 von der Umformungsvorrichtung 1 erreicht werden.

Mit einer Übergabeeinheit 42 werden die expandierten Kunststoffbehältnisse an eine Befüllungseinrichtung 40 übergeben und von dieser Befüllungseinrichtung 40 anschließend über eine weitere Transporteinheit 44 abgeführt. Dabei befindet sich auch die Befüllungseinrichtung 40 innerhalb des besagten Reinraums 6. Auch im Falle der Befüllungseinrichtung wäre es möglich, dass nicht die gesamte Befüllungseinrichtung 40 mit beispielsweise einem Reservoir für ein Getränk vollständig innerhalb des Reinraums 6 angeordnet sind, sondern auch hier lediglich diejenigen Bereiche, in denen tatsächlich die Behältnisse geführt werden. Insoweit könnte auch die Befüllungseinrichtung in ähnlicher Weise aufgebaut sein wie die Vorrichtung 1 zum Umformen von Kunststoffvorformlingen 10.

Wie erwähnt, ist der Reinraum 6 im Bereich der Vorrichtung 1 auf einen geringst möglichen Bereich reduziert, nämlich im Wesentlichen auf die Blasstationen 8 selbst. Durch diese kleinbauende Gestaltung des Reinraums 6 ist es leichter und schneller möglich, einen Reinraum überhaupt herzustellen und auch die Sterilhaltung in der Betriebsphase ist weniger aufwändig. Auch wird weniger Sterilluft benötigt, was zu kleineren Filteranlagen führt und auch die Gefahr von unkontrollierter Wirbelbildung wird reduziert.

Figur 2 zeigt eine Detaildarstellung der Vorrichtung 1 im Bereich einer Blasstation 8. Eine Vielzahl derartiger Blasstationen 8 wird mit einer Transporteinrichtung 2 drehend um eine Achse X bewegt. Die Blasstation 8 ist, wie in Figur 2 ersichtlich, innerhalb des Reinraums 6, der hier kanalartig ausgebildet ist, geführt. Dieser Reinraum 6 wird abgeschlossen durch eine bewegliche Seitenwand 16 und einen einteilig mit dieser Seitenwand 16 ausgebildeten Deckel 17. Diese Seitenwand 16 und der Deckel 17 drehen dabei mit der Blasstation 8 mit.

Das Bezugszeichen 18 bezieht sich auf eine weitere Wandung, welche den Reinraum 16 begrenzt. Diese Wandung 18 ist hier eine außen liegende Wandung, welche stationär angeordnet ist. Zwischen dem Deckel 17 und der Wandung 18 ist eine Dichtungseinrichtung 25 vorgesehen, welche die gegeneinander beweglichen Elemente 17 und 18 gegeneinander abdichtet, beispielsweise, wie oben erwähnt, unter Verwendung eines Wasserschlosses. Der untere Bereich der Wandung 18 ist fest und abdichtend an einem Boden 13 angeordnet. Innerhalb des Reinraums 6 und hier unmittelbar an der Wandung 16 anliegend ist ein Träger 26 vorgesehen, der sich ebenfalls drehend bewegt und an dem wiederum eine Halteeinrichtung 23 vorgesehen ist, welche die Blasstation 8 hält.

Das Bezugszeichen 11 bezieht sich auf eine Folgeeinrichtung, welche von einer Führungskurve 9 betätigt werden kann, um die Blasstation auf ihrem Weg durch den Reinraum 6 zu öffnen und zu schließen, um insbesondere den Kunststoffvorformling in die Blasstation einzulegen und um ihn auch wieder zu entnehmen. Dabei ist eine Führungskurve 9 auch innerhalb des Reinraums 6 angeordnet. Es wäre jedoch beispielsweise auch möglich, etwa bereits einen Abschnitt 19 unterhalb der einzelnen Blasstationen 8 aus dem Reinraum 6 herauszuführen.

Die Transporteinrichtung 2 kann noch weitere Elemente aufweisen, welche oberhalb des Reinraums 6 angeordnet sind.

Der Träger 26 ist dabei fest auf einem Haltekörper 29 angeordnet und dieser Haltekörper ist wiederum beweglich gegenüber dem Boden 13. Dabei bezieht sich das Bezugszeichen 27 auf eine weitere Dichtungseinrichtung, welche auch in diesem Bereich eine Abdichtung der gegeneinander beweglichen Bereiche 13 und 29 bewirkt.

Das Bezugszeichen 5 bezieht sich auf eine Reckstange, welche gegenüber der Blasstation beweglich ist, um die Kunststoffvorformlinge 10 in ihrer Längsrichtung zu recken. Dabei ist auf dem Deckel 17 hier ein Schlitten 12 angeordnet, demgegenüber die Reckstange in der Richtung Y beweglich ist. Das Bezugszeichen 14 bezieht sich auf eine weitere Halterung für diesen Schlitten 12 der Reckstange 5.

Man erkennt, dass bestimmte Bereiche der Reckstange während des Blasvorgangs sowohl außerhalb des Reinraums 6 als auch innerhalb des Reinraums sind. Zu diesem Zweck ist es möglich, außerhalb des Reinraums 6 bzw. oberhalb des Schlittens 12 eine Schutzeinrichtung wie einen Faltenbalg vorzusehen, der die Reckstange 5 umgibt, so dass kein Bereich der Reckstange 5 unmittelbar mit der Außenumgebung in Berührung kommt.

Die oben erwähnte Heizeinrichtung zum Erwärmen der Kunststoffvorformlinge ist bevorzugt ebenfalls aseptisch ausgeführt. Dies bedeutet, dass die Kunststoffvorformlinge bereits im Bereich der Heizeinrichtung 30, anders als in Fig. 1 gezeigt, durch einen Reinraum geführt werden können und sich dieser Reinraum beispielsweise durchgehend über die Blasmaschine bis zu dem Füller erstreckt. Dabei ist es möglich, dass die vollständige Heizeinrichtung 30 innerhalb eines Sterilraums angeordnet ist, es wäre jedoch auch möglich, dass auch hier insbesondere der Bereich, in dem die Kunststoffvorformlinge transportiert werden, gegenüber der Umgebung als Sterilraum gekapselt ist. So wäre es beispielsweise möglich, dass die Kunststoffvorformlinge mittels Dornen transportiert werden, die in ihre Mündung eingreifen und die Dornen dabei durch eine Wandung in einen Reinraum hineinragen. Dieser Reinraum könnte ebenfalls mit einem Überdruck beaufschlagt werden, so dass keine Umgebungsluft in diesen Reinraum eindringen kann.

Die Heizeinrichtung könnte dabei als Infrarotheizeinrichtung ausgestaltet sein, wie in Fig. 1 dargestellt. Vorteilhaft wird jedoch hier als Heizeinrichtung eine Mikrowellenheizeinrichtung verwendet. Derartige Mikrowellenheizeinrichtungen zum Erwärmen von Kunststoffvorformlingen sind aus dem Stand der Technik an sich bekannt. Dabei könnte eine Vielzahl von Mikrowellenheizstationen beispielsweise an einem Trägerrad angeordnet sein. Über Schleusen könnten die Kunststoffvorformlinge diesen einzelnen Heizstationen zugeführt werden. Aufgrund der Ausführung mit einzelnen Heizstationen eignet sich eine mikrowellenbasierte Heizeinrichtung in besonderer Weise für die Kombination mit Sterilräumen.

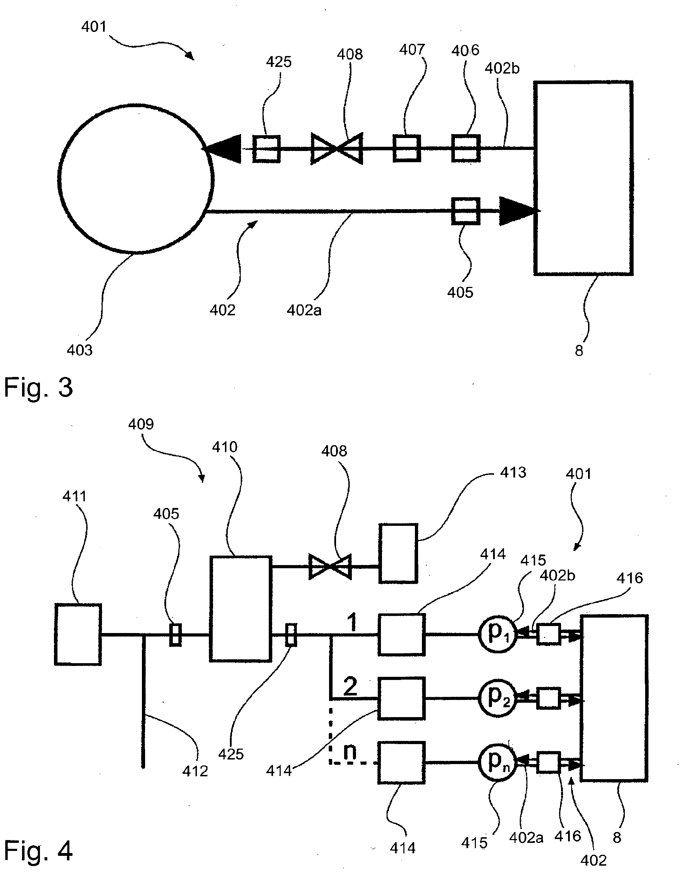

Fig. 3 zeigt eine schematische Darstellung einer Vorrichtung 401 mit Verbindungsleitungen 402, 402a, 402b zwischen einem Reservoir 403 und einer einzelnen Blasstation 8. Dabei kennzeichnet der untere Pfeil eine Gaszuleitung 402a, durch welche Gas eines vorgegebenen Druckes Pₙ von einem Reservoir 403 zu einer Blasstation 8 geführt werden kann. Zur Vermeidung von Verunreinigungen in der Blasstation 8 ist in dieser Zugasleitung 402a ein Sterilfilter 405 angeordnet. Durch diesen werden eventuell vorhandene mikrobielle Verunreinigungen zurückgehalten, bevor sie mit den Kunststoffvorformlingen bzw. Kunststoffbehältnissen in Kontakt kommen können. In einer Gasableitung 402b, welche durch den oberen Pfeil dargestellt ist, ist ein Katalysator 406 angeordnet.

Im Fall einer notwendigen Desinfektion der Blasform oder Blasstation 8 kann mit diesem Katalysator 406 eventuell in der Blasstation 8 verbliebenes oxidatives Desinfektions-/Sterilisationsmittel unschädlich gemacht werden. Im Fall von H₂O₂ als Desinfektions-/Sterilisationsmittel erfolgt die Spaltung in H₂O und O₂. Stromabwärts bezüglich des Katalysators 406 ist eine Messvorrichtung 407 angeordnet, welche die Qualität des durchströmenden Gases überprüft. Abhängig von den ermittelten Messwerten bezüglich der Gasqualität kann eine weiter stromabwärts angeordnete Absperrvorrichtung 408 gesteuert werden, welche die Gasleitung 402b verschließen kann. Dadurch wird beispielsweise bei einer Rückführung des Gases von einer Blasstation 8 in das Reservoir 403 eine Anreicherung des oxidativen Desinfektions-/Sterilisationsmittels innerhalb der Leitungen oder innerhalb des Reservoirs 403 vermieden.

Um auch mikrobielle Kontaminationen des Reservoirs 403 zu vermeiden, ist in der gezeigten Ausführungsform ein weiterer Sterilfilter 425 im Leitungssystem 402 angeordnet. Um mikrobielle Kontaminationen des Reservoirs 403 zu vermeiden, wird die zurück in das Reservoir 403 geleitete Gas vor dem Eintritt in das Reservoir 403 steril gefiltert. Verunreinigungen, die beispielsweise in einem Kunststoffvorformling in die Blasmaschine eingeschleppt werden, und durch die Druckgasbeaufschlagung aufgewirbelt werden, können so davon abgehalten werden, in den Druckgaskreislauf zu gelangen.

Fig. 4 zeigt eine schematische Darstellung eines Systems von Leitungen 409 mit verschiedenen Gasdrücken Pₙ innerhalb einer Blasmaschine. In dem gezeigten System 409 wird ein Gasverteiler 410 von einer zentralen nicht gezeigten Druckgaserzeugungseinheit mittels einer Druckgaszuleitung 411 mit Druckgas gespeist. Die Zuführung des Druckgases erfolgt bereits steril, was durch die Einspeisung eines Sterilisationsgases 412 sowie einer Sterilfilterung mittels eines Filters 405 gewährleistet wird. Ausgehend von dem Gasverteiler 410 werden sowohl Gasverbraucher 413 ohne direkten Kontakt mit Produkt oder Verpackungsmaterial als auch Druckminderer 414 zur Versorgung der Blasstationen 8 mit Druckgas versorgt. Die Leitung zu Gasverbrauchern 413 ohne direkten Kontakt mit Produkt oder Verpackungsmaterial wird über eine Absperrvorrichtung 408 wie beispielsweise einem Ventil reguliert. Die Zuleitung zu den Druckminderen 414 erfolgt erneut durch einen Sterilfilter 425. Stromabwärts der Druckminderer 414 sind verschiedene Druckgasreservoirs 415 wie insbesondere Ringkanäle angeordnet, die wiederum mit den einzelnen Blasstationen 8 in Kontakt stehen. Die einzelnen Reservoirs 415 können dabei sowohl gleiche als auch voneinander verschiedene Gasdrücke Pₙ aufweisen. Die Verbindung 402 zwischen den einzelnen Reservoirs 403 und den einzelnen Blasstationen 8 kann analog der in Fig. 1 gezeigten Ausführungsform erfolgen. Es ist jedoch auch abweichend von dem gezeigten Beispiel möglich, die von der Blasstation 8 zurückgeführte Gas nicht demjenigen Reservoir 415 zuzuführen aus dem sie der Blasstation 8 zugeführt wurde, sondern einem anderen Reservoir 415, das Gas eines geringeren Druckes bereithält. Beispielsweise kann das Abgas aus einem Prozessschritt, welcher mit dem höchsten Druck P₁ durchgeführt wurde dem Reservoir 415 mit dem Druck P₂ zugeführt werden. Eine derartige Quervernetzung der unterschiedlichen Reservoirs 403 erhöht jedoch die Gefahr einer Kontamination großer Bereiche der Blasmaschine, weshalb vorteilhaft entsprechende Sicherheitsmaßnahmen vorgesehen sind. Die in der dargestellten schematischen Zeichnung gezeigten Gasaufbereitungseinrichtungen 416 können daher sowohl in der Gaszuleitung 402a als auch in der Gasableitung 402b angeordnet sein.

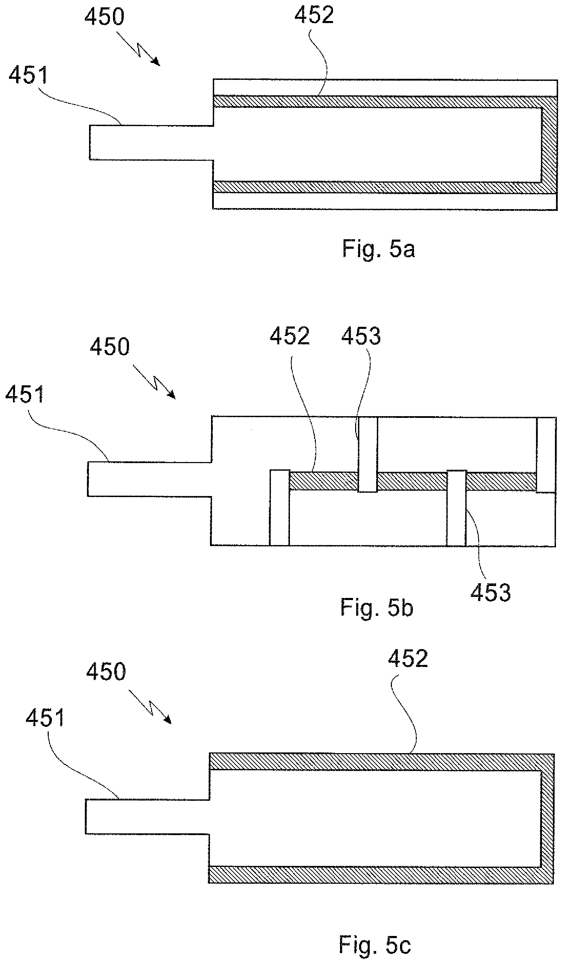

In den Fig. 5a - 5c sind verschiedenen Ausführungsvarianten eines Schalldämpfers 450 mit integriertem Katalysator gezeigt. In allen beispielhaft gezeigten Ausführungsvarianten ist im linken Bereich eine Zuleitung bzw. Anschlussvorrichtung gezeigt, über welche Gas in den Schalldämpfer 450 eingeleitet werden kann. In Fig. 5a ist schematisch eine Ausführungsform gezeigt, in der die Katalysatorschicht 452 auf der Innenseite des Schalldämpfers 450 aufgebracht wurde. Als katalysatortragende Schicht kann auf aus dem Stand der Technik bekannte Elemente zurückgegriffen werden. Beispielsweise können poröse Sinterformteile oder Keramiken als Basis dienen, auf die der Katalysator aufgebracht wird. Die äußere Schicht besteht aus einem Schalldämmenden Material, beispielsweise aus Polypropylen oder anderen. Abgase aus dem Schalldämpfer können entweder über die Außenfläche in die Umgebung abgegeben werden oder über eine separate Abgasleitung abgeführt werden.

Fig. 5b zeigt eine Variante eines Schalldämpfers 450 mit integriertem Katalysator, in der der Gasfluss im Inneren des Schalldämpfers 450 über Wände 453 in verschiedenen Abschnitten jeweils umgelenkt wird. Dadurch wird der Gasstrom beim Durchlaufen des Schalldämpfers mehrfach durch eine Katalysatorschicht 452 geführt. In Zwischenräumen befinden sich die Katalysatorschichten Es sind auch andere Anordnungen von Wänden 453 und Katalysatoren 452 möglich. Beispielsweise könnte der gasdurchlässige Katalysator 452 jeweils in Verlängerung der gasundurchlässigen Wände 453 angeordnet sein. Die Anzahl der Wände 453 kann den jeweiligen Erfordernissen angepasst werden und liegt üblicherweise zwischen 1 und 100, bevorzugt zwischen 2 und 20. In einem derartigen Schalldämpfer können durch genau definierte Querschnitte und Formen die Geräuschkulisse und die Durchflussmenge beeinflusst werden.
In Fig. 5c ist eine Variante eines Schalldämpfers 450 mit integriertem Katalysator gezeigt, in der das Katalysatormaterial 452 so aufgebaut ist, dass es gleichzeitig auch schalldämpfende Eigenschaften aufweist. Beispielsweise kann es schwammartig, als mehrlagige Schicht aus engmaschigen Netzen oder in anderen ähnlichen Anordnungen mit großer innerer Oberfläche und somit vielen Schallrefletionsflächen aufgebaut sein. Das schalldämpfende Material ist durch ein schalldämpfendes und gleichzeitig katalytisches Material ersetzt worden. Der Katalysator kann in Form von Granulat, Dünnschicht auf porösem Material oder Sinterform oder in in anderen Formen direkt aus einem katalytischen Material ausgeführt sein. Beim Durchlaufen dieser Schicht wird der Schall vielfach in verschiedene Richtungen reflektiert und die Schallwellen laufen aus. Gleichzeitig kommt auch mit dem Gasstrom transportiertes Desinfektions/Sterilisationsmittel mit dem Katalysator 452 in Kontakt und kann gespalten werden.

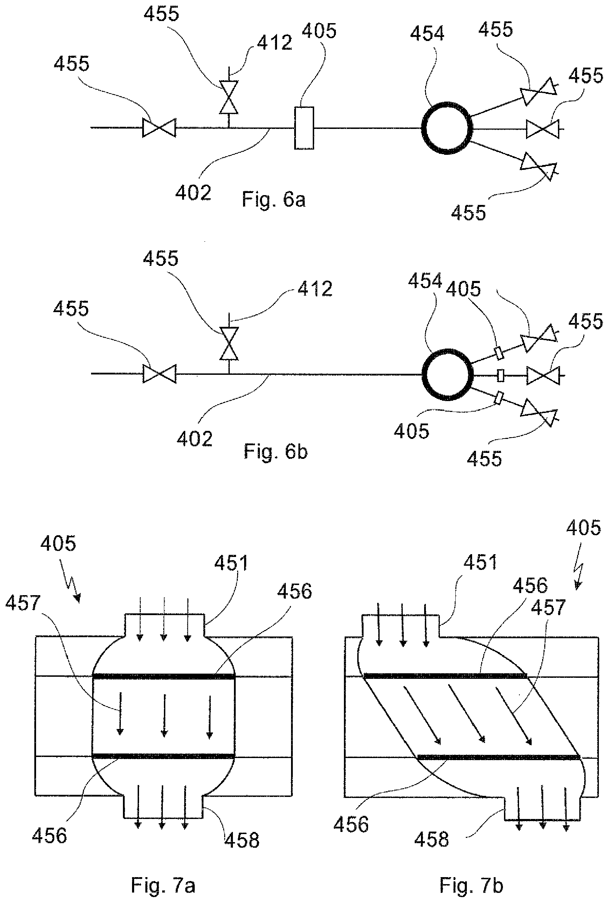

Fig. 6a zeigt die erfindungsgemäße Variante der möglichen verschiedenen Anordnungen des / der Sterilfilter/s 405 im Leitungssystem. Bei der in Fig. 6a dargestellten Variante ist ein zentraler Sterilfilter 405 im Leitungssystem 402 angeordnet. Diese befindet sich bevorzugt an einer Position stromabwärts der Einspeisung des Sterilisationsgases 412, jedoch stromaufwärts des Ringkanals 454. Über verschiedene Ventile 455 können die einzelnen Gasströme reguliert werden. Ein sich bevorzugt ebenfalls Stromabwärts der Einspeisung des Sterilisationsgases 412 befindlicher Drehverteiler ist in den Fig. 4a und 4b nicht gezeigt.

In Fig. 6b ist die alternative erfindungsgemäße Variante der Anordnung von Sterilfiltern 405 im Leitungssystem 402 gezeigt. In diesem Beispiel sind mehrere Sterilfilter 405 stromabwärts der Einspeisung des Sterilisationsgases 412 jedoch im Gegensatz zu der in Fig. 6a gezeigten Ausführungsform auch stromabwärts des Ringkanals 454 und stromaufwärts bezüglich der jeweiligen (nicht gezeigten) Blasstationen angeordnet. Stromabwärts der Sterilfilter 405 sind Ventile 455 angeordnet, welche die Gaszuführung zu den einzelnen Blasstationen regulieren.
Fig. 7a zeigt einen Sterilfilter 405 nach dem Stand der Technik. Die Filtermembranen 456 sind in Strömungsrichtung des Gases 457 direkt hintereinander angeordnet. Das Zentrum jeder Membran liegt auf einer Achse mit den Zentren der Gaszuleitung 451 und der Gasableitung 458. Nachteil dieser Ausführungsform ist, dass bei einem überwiegend laminaren Strömungsverlauf die Strömungsgeschwindigkeit des Gases im Zentrum größer ist als in Randbereichen, wodurch es zu einer stärkeren Verschmutzung des Zentrums jeder der Membranen kommen kann. Außerdem werden die Randbereiche der Membranen, da hier der Gasstrom geringer ist, mit geringeren Mengen an Desinfektions-/Sterilisationsmittel beaufschlagt. Dies kann dazu führen, dass dort keine ausreichende Sterilisation erfolgt. Soll dies dennoch sichergestellt werden, sind große Mengen an Desinfektions-/Sterilisationsmittel notwendig.
In der in Fig. 7b gezeigten Variante eines Sterilfilters 405 sind die Filtermembranen 456 in Strömungsrichtung des Gases 457 gegenüber einander quer zur Längsrichtung (bzw. Strömungsrichtung des Gases) des Sterilfilters 405 versetzt. Auch die Anschlüsse für Zu- und Abluft sind außermittig gesetzt. Dadurch wird das Gas zwangsweise abgelenkt, wodurch ein turbulenter Strömungsverlauf hervorgerufen werden kann. Dadurch kann eine gleichmäßigere Verteilung der Strömungsgeschwindigkeiten über die gesamte Membranfläche erreicht werden. Dies resultiert einerseits in einer gleichmäßigeren Ablagerung von Verunreinigungen auf der jeweiligen Filtermembran 456, andererseits auch in einer homogeneren Verteilung des Desinfektions-/Steritisationsmittels über die gesamte Filtermembran 456. Außerdem führt dies zu einer Vergrößerung der Luftdurchsetzten (Membran-) Fläche. Durch die Optimierung der Luftverteilung im Membranfilter kannd as Filtermaterial besser ausgenutzt werden. So ist es auch bei geringerem Desinfektions-/Sterilisationsmitteleinsatz möglich, die Randbereiche der Filtermembranen 456 ausreichend zu desinfizieren/sterilisieren. Zusammenfassend wird durch die erfindungsgemäße Vorrichtung sowie das erfindungsgemäße Verfahren eine Möglichkeit geschaffen, um insbesondere zurück gewonnene Druckluft vor dem erneuten Gebrauch wieder steril zu filtern und somit die Sterilität des Gesamtsystems zu jedem Zeitpunkt während der Produktion aufrecht zu erhalten.

### Bezugszeichenliste

- 1: Umformungsvorrichtung
- 2: Transportrad
- 5: Reckstange
- 6: Reinraum
- 8: Blasstation
- 10: Kunststoffvorformlinge, Behältnis
- 11: Folgeeinrichtung
- 12: Schlitten
- 13: Boden
- 14: Halterung
- 15: Knick
- 16: Seitenwand
- 17: Deckel
- 18: Wandung
- 19: Abschnitt
- 20: Kunststoffbehältnis
- 22: Zuführeinrichtung
- 23: Halteeinrichtung
- 24: Abführeinrichtung
- 25: Dichtungseinrichtung
- 26: Träger
- 27: Dichtungseinrichtung
- 29: Haltekörper
- 30: Heizeinrichtung
- 31: Heizelemente
- 32: Sterilisationseinrichtung
- 34: Transporteinrichtung
- 36: Übergabeeinheit
- 37: Transportrad
- 40: Befüllungseinrichtung
- 42: Übergabeeinheit
- 44: Transporteinheit
- 50: Anlage
- 401: Vorrichtung
- 402: Gasleitung
- 402a: Gaszuleitung
- 402b: Gasableitung
- 403: Reservoir
- 405: Sterilfilters
- 406: Katalysator
- 407: Messvorrichtung
- 408: Absperrvorrichtung
- 409: System von Leitungen
- 410: Gasverteiler
- 411: Druckgaserzeugungseinheit
- 412: Sterilisationsgaszuleitung
- 413: Gasverbraucher ohne direkten Kontakt mit Produkt oder Verpackungsmaterial
- 414: Druckminderer
- 415: Druckgasreservoir mit Gasdruck Pₙ
- 416: Gasaufbereitungseinrichtung
- 425: Sterilfilter
- 450: Schalldämpfer mit Katalysator
- 451: Zuleitung / Anschlussvorrichtung
- 452: Katalysatorschicht
- 453: Wand
- 454: Ringleitung
- 455: Ventil
- 456: Filtermembran
- 457: Strömungsrichtung
- 458: Ableitung / Anschlussvorrichtung
- Pₙ: Gasdruck
- L: Linie

## Patentansprüche

1. Vorrichtung (401) zur Reinigung von Gas in Blasmaschinen zum Umformen von Kunststoffvorformlingen zu Kunststoffbehältnissen,
wobei
in mindestens einer Gasleitung (402, 402a, 402b), welche eine Gaszuleitung (402a) oder eine Gasableitung (402b) einer Blasstation (8) ist, mindestens eine Gasaufbereitungseinrichtung (416) angeordnet ist, welche in der Lage ist, Gas steril zu filtern und/oder eine Menge eines in dem durchströmenden und/oder vorbeiströmenden Gas enthaltenen oxidativen Desinfektions-/Sterilisationsmittels zu reduzieren,
- mindestens eine Gasaufbereitungseinrichtung (416) ein Filter (405) ist, welcher in der Lage ist, Gas steril zu filtern und dieser Filter (405) in einer Gasleitung (402, 402a) angeordnet ist, durch welche Druckgas zu einer Blasstation (8) zuführbar ist, **dadurch gekennzeichnet, dass**
- der Filter (405) als ein zentraler Sterilfilter (405) im Leitungssystem (402) angeordnet ist, wobei dieser Sterilfilter (405) an einer Position stromabwärts einer Einspeisung eines Sterilisationsgases (412), jedoch stromaufwärts eines Ringkanals (454) angeordnet ist,
wobei über verschiedene Ventile (455) die einzelnen Gasströme reguliert werden können,oder
die Gasaufbereitungseinrichtung (416) mehrere Sterilfilter (405) umfasst, die stromabwärts einer Einspeisung eines Sterilisationsgases (412) jedoch auch stromabwärts eines Ringkanals (454) und stromaufwärts bezüglich jeweiliger Blasstationen angeordnet sind, wobei stromabwärts der Sterilfilter (405) Ventile (455) angeordnet sind, welche die Gaszuführung zu den einzelnen Blasstationen regulieren.

2. Vorrichtung (401) nach Anspruch 1,
**dadurch gekennzeichnet, dass**
mindestens eine Gasaufbereitungseinrichtung (416) einen Filter (425) umfasst, welcher in der Lage ist, Gas steril zu filtern und dieser Filter (425) in einer Gasleitung (402b) angeordnet ist, durch welche Druckgas von einer Blasstation (8) abführbar ist.

3. Vorrichtung (401) nach einem der vorangegangenen Ansprüche,
**dadurch gekennzeichnet, dass**
mindestens eine Gasaufbereitungseinrichtung (416) einen Katalysator (406) umfasst, welcher dazu geeignet ist, in durchströmendem und/oder vorbeiströmendem Gas enthaltene oxidative Desinfektions-/Sterilisationsmittel in ihrer Menge zu reduzieren und dieser Katalysator (406) in einer Gasleitung (402b) angeordnet ist, durch welche Gas von einer Blasstation (8) abführbar ist.

4. Vorrichtung (401) nach Anspruch 3,
**dadurch gekennzeichnet, dass**
der mindestens eine Katalysator (406) ein Katalysator (406) ist, welcher dazu geeignet ist, die Menge an H₂O₂ in dem Gas zu reduzieren.

5. Vorrichtung (401) nach einem der vorangegangenen Ansprüche,
**dadurch gekennzeichnet, dass**
der mindestens eine Katalysator (406) ein Metall umfasst, welches bevorzugt aus einer Gruppe ausgewählt ist, die Platin, Palladium, Nickel, Gold, Silber, Kupfer, Rhodium, Kobalt, Osmium, Eisen, Chrom, Vanadium, Zirkonium, Hafnium, Cer, Samarium, Zink, Mangan und Kombinationen hiervon und dergleichen umfasst.

6. Vorrichtung (401) nach einem der vorangegangenen Ansprüche,
**dadurch gekennzeichnet, dass**
die Vorrichtung mindestens einen Schalldämpfer (450) aufweist und dieser Schalldämpfer wenigstens einen Katalysator aufweist, wobei der Katalysator (406) mit einem Schalldämpfer (450) kombiniert und/oder in einen Schalldämpfer (450) integriert ist.

7. Vorrichtung (401) nach einem der vorangegangenen Ansprüche,
**dadurch gekennzeichnet, dass**
der mindestens eine Filter (405, 425) zum Sterilfiltern von Gas Porengrößen von weniger als 50 µm, bevorzugt von weniger als 5 µm, besonders bevorzugt von weniger als 0,5 µm aufweist.

8. Vorrichtung (401) nach einem der vorangegangenen Ansprüche,
**dadurch gekennzeichnet, dass**
in mindestens einer der Gasleitungen (402, 402a, 402b) stromabwärts bezüglich mindestens eines Filters (405, 425) oder eines Katalysators (406) eine Messvorrichtung (407) angeordnet ist, welche dazu geeignet ist, die Reinigungsleistung des stromaufwärts angeordneten Filters (405, 425) oder Katalysators (406) zu messen.

9. Vorrichtung (401) nach Anspruch 8,
**dadurch gekennzeichnet, dass**
in mindestens einer der Gasleitungen (402, 402a, 402b) stromabwärts bezüglich mindestens einer Messvorrichtung (407) eine Absperrvorrichtung (408) zum Verschließen der Gasleitung (402) angeordnet ist.

10. Vorrichtung (401) nach Anspruch 9,
**dadurch gekennzeichnet, dass**
die Absperrvorrichtung (408) zum Verschließen der Gasleitung (402, 402a, 402b) mit mindestens einer Steuereinrichtung verbunden ist, welche dazu geeignet ist, die Absperrvorrichtung (408) abhängig von den von der Messvorrichtung (407) ermittelten Werten zu verschließen oder zu öffnen.

11. Vorrichtung (401) nach wenigstens einem der vorangegangenen Ansprüche,
**dadurch gekennzeichnet, dass**
die Vorrichtung (401) einen Reinraum (6) aufweist, durch welchen die Behältnisse transportiert werden.

12. Verfahren zur Reinigung von Gas in Blasmaschinen zum Umformen von Kunststoffvorformlingen zu Kunststoffbehältnissen,
wobei
das Gas durch mindestens eine Gasaufbereitungseinrichtung (405, 406, 416) geleitet wird, welche das Gas bevorzugt mittels eines Sterilfilters (405) sterilisiert und/oder mittels eines Katalysators (406) Restmengen von oxidativen Desinfektions-/Sterilisationsmitteln in dem Gas reduziert, wobei
- mindestens eine Gasaufbereitungseinrichtung (416) ein Filter (405) ist, welcher in der Lage ist, Gas steril zu filtern und dieser Filter (405) in einer Gasleitung (402, 402a) angeordnet ist, durch welche Druckgas zu einer Blasstation (8) zuführbar ist, **dadurch gekennzeichnet, dass**
- der Filter (405) als ein zentraler Sterilfilter (405) im Leitungssystem (402) angeordnet ist, wobei dieser Sterilfilter (405) an einer Position stromabwärts einer Einspeisung eines Sterilisationsgases (412), jedoch stromaufwärts eines Ringkanals (454) angeordnet ist,
wobei über verschiedene Ventile (455) die einzelnen Gasströme reguliert werden, oder
- die Gasaufbereitungseinrichtung (416) mehrere Sterilfilter (405) umfasst, die stromabwärts einer Einspeisung eines Sterilisationsgases (412) jedoch auch stromabwärts eines Ringkanals (454) und stromaufwärts bezüglich jeweiliger Blasstationen angeordnet sind, wobei stromabwärts der Sterilfilter (405) Ventile (455) angeordnet sind, welche die Gaszuführung zu den einzelnen Blasstationen regulieren.

13. Verfahren nach Anspruch 12,
**dadurch gekennzeichnet, dass**
der in einem Leitungssystem der Blasmaschinen angeordnete Sterilfilter (405) mittels eines oxidativen Desinfektions-/Sterilisationsmittels gereinigt wird, welches auch für eine Desinfektion/Sterilisation von Kunststoffvorformlingen und/oder Kunststoffbehältnissen verwendet wird.

## Claims

1. An apparatus (401) for cleaning gas in blow moulding machines for shaping plastics material pre-forms to form plastics material containers, wherein
at least one gas-processing device (416), which is capable of filtering the gas in a sterile manner and/or of reducing a quantity of an oxidative disinfecting / sterilizing agent contained in the gas flowing through and/or flowing past, is arranged in at least one gas line (402, 402a, 402b) which is a gas supply line (402a) or a gas removal line (402b) of a blowing station (8),
- at least one gas-processing device (416) is a filter (405) which is capable of filtering gas in a sterile manner and this filter (405) is arranged in a gas line (402, 402a) through which compressed gas can be fed to a blowing station (8), **characterized in that**
- the filter (405) is arranged as a central sterile filter (405) in the line system (402), wherein this sterile filter (405) is arranged at a position downstream of a feed of a sterilizing gas (412) but upstream of an annular channel (454),
wherein the individual gas streams can be regulated via different valves (455) or
the gas-processing device (416) comprises a plurality of sterile filters (405) which are arranged downstream of an feed of a sterilizing gas (412) but also downstream of an annular channel (454) and upstream of respective blowing stations, wherein downstream of the sterile filters (405) valves (455) are arranged, which regulate the gas supply to the individual blowing stations.

2. An apparatus (401) according to claim 1,
**characterized in that**
at least one gas-processing device (416) comprises a filter (425) which is capable of filtering gas in a sterile manner and this filter (425) is arranged in a gas line (402b) through which compressed gas is capable of being removed from a blowing station (8).

3. An apparatus (401) according to any one of the preceding claims,
**characterized in that**
at least one gas-processing device (416) comprises a catalyst (406) which is suitable for reducing oxidative disinfecting / sterilizing agents contained in the gas flowing through and/or flowing past in their quantity and this catalyst (406) is arranged in a gas line (402b) through which gas is capable of being removed from a blowing station (8).

4. An apparatus (401) according to claim 3,
**characterized in that**
the at least one catalyst (406) is a catalyst (406) which is suitable for reducing the quantity of H₂O₂ in the gas.

5. An apparatus (401) according to any one of the preceding claims,
**characterized in that**
the at least one catalyst (406) comprises a metal which is preferably selected from a group which consists of platinum, palladium, nickel, gold, silver, copper, rhodium, cobalt, osmium, iron, chromium, vanadium, zirconium, hafnium, cerium, samarium, zinc, manganese and combinations thereof and the like.

6. An apparatus (401) according to any one of the preceding claims,
**characterized in that**
the apparatus has at least one silencer (450), and this silencer has at least one catalyst, wherein the catalyst (406) is combined with a silencer (450) and/or is integrated in a silencer (450).

7. An apparatus (401) according to any one of the preceding claims,
**characterized in that**
the at least one filter (405, 425) for the sterile filtering of gas has pore sizes of less than 50 µm, preferably of less than 5 µm, and in a particularly preferred manner of less than 0.5 µm.

8. An apparatus (401) according to any one of the preceding claims,
**characterized in that**
a measuring appliance (407), which is suitable for measuring the cleaning performance of a filter (405, 425) or a catalyst (406) arranged upstream, is arranged in at least one of the gas lines (402, 402a, 402b) downstream with respect to at least one filter (405, 425) or catalyst (406).

9. An apparatus (401) according to claim 8,
**characterized in that**
a shut-off apparatus (408) for closing the gas line (402) is arranged in at least one of the gas lines (402, 402a, 402b) downstream with respect to at least one measuring appliance (407).

10. An apparatus (401) according to claim 9,
**characterized in that**
the shut-off apparatus (408) for closing the gas line (402, 402a, 402b) is connected to at least one control device which is suitable for closing or opening the shut-off apparatus (408) in a manner dependent upon the values determined by the measuring appliance (407).

11. An apparatus (401) according to any one of the preceding claims,
**characterized in that**
the apparatus (401) has a clean room (6) through which the containers are conveyed.

12. A method of cleaning gas in blow moulding machines for shaping plastics material pre-forms to form plastics material containers,
wherein
the gas is conveyed through at least one gas-processing device (405, 406, 416) which sterilizes the gas preferably by means of a sterile filter (405) and/or reduces residual quantities of oxidative disinfecting / sterilizing agents in the gas by means of a catalyst (406), wherein
- at least one gas-processing device (416) is a filter (405) which is capable of filtering gas in a sterile manner and this filter (405) is arranged in a gas line (402, 402a) through which compressed gas can be fed to a blowing station (8), **characterized in that**
- the filter (405) is arranged as a central filter (405) in the line system (402), wherein this sterile filter (405) is arranged at a position downstream of a feed of a sterilizing gas (412) but upstream of an annular channel (454),
wherein the individual gas streams can be regulated via different valves (455)
or
- the gas-processing device (416) comprises a plurality of sterile filters (405) which are arranged downstream of a feed of a sterilizing gas (412) but also downstream of an annular channel (454) and upstream of respective blowing stations wherein downstream of the sterile filters (405) valves (455) are arranged which regulate the gas supply of the individual blowing stations.

13. A method according to claim 12,
**characterized in that**
the sterile filter (405) arranged in a line system of the blow moulding machines is cleaned by means of an oxidative disinfecting / sterilizing agent which is also used for the disinfection / sterilization of plastics material pre-forms and/or plastics material containers.

## Revendications

1. Dispositif (401) de nettoyage de gaz dans des machines de soufflage servant à transformer des ébauches en matière plastique en récipients en matière plastique,
dans lequel
au moins un système de traitement de gaz (416), lequel étant apte à effectuer un filtrage du gaz de manière stérile et/ou de réduire une quantité d'un désinfectant/stérilisant oxydatif contenu dans le gaz circulant et/ou s'écoulant, étant agencé dans au moins une conduite de gaz (402, 402a, 402b), laquelle étant une conduite d'amenée de gaz (402a) ou une conduite d'évacuation de gaz (402b) d'une station de soufflage (8),
- au moins un système de traitement de gaz (416) étant un filtre (405) qui est apte à effectuer un filtrage du gaz de manière stérile et ledit filtre (405) étant agencé dans une conduite de gaz (402, 402a) à travers laquelle un gaz comprimé peut être amené à une station de soufflage (8), **caractérisé en ce que**
- le filtre (405) utilisé comme filtre stérile central (405) est agencé dans le système de conduite (402), dans lequel ledit filtre stérile (405) est agencé dans une position en aval d'une alimentation d'un gaz de stérilisation (412), mais en amont d'un canal annulaire (454),
dans lequel les différents courants de gaz peuvent être régulés par différentes soupapes (455), ou
le système de traitement de gaz (416) comporte plusieurs filtres stériles (405), qui sont agencés en aval d'une alimentation d'un gaz de stérilisation (412) mais également en aval d'un canal annulaire (454) et en amont des stations de soufflage respectives, dans lequel des soupapes (455), régulant l'amenée de gaz aux différentes stations de soufflage, sont agencées en aval des filtres stériles (405).

2. Dispositif (401) selon la revendication 1,
**caractérisé en ce que**
au moins un système de traitement de gaz (416) comprend un filtre (425), lequel est apte à effectuer un filtrage du gaz de manière stérile et ledit filtre (425) est agencé dans une conduite de gaz (402b) à travers laquelle le gaz comprimé peut être évacué d'une station de soufflage (8).

3. Dispositif (401) selon l'une quelconque des revendications précédentes,
**caractérisé en ce que**
au moins un système de traitement de gaz (416) comprend un catalyseur (406), lequel est conçu pour réduire la quantité d'un désinfectant/stérilisant oxydatif contenu dans le gaz circulant et/ou s'écoulant et ce catalyseur (406) est agencé dans une conduite de gaz (402b) à travers laquelle le gaz peut être évacué d'une station de soufflage (8).

4. Dispositif (401) selon la revendication 3,
**caractérisé en ce que**
ledit au moins un catalyseur (406) est un catalyseur (406) qui est adapté à réduire la quantité de H₂O₂ dans le gaz.

5. Dispositif (401) selon l'une quelconque des revendications précédentes,
**caractérisé en ce que**
ledit au moins un catalyseur (406) comporte un métal qui est choisi de préférence dans un groupe comprenant le platine, le palladium, le nickel, l'or, l'argent, le cuivre, le rhodium, le cobalt, l'osmium, le fer, le chrome, le vanadium, le zirconium, le hafnium, le cérium, le samarium, le zinc, le manganèse et des combinaisons de ces derniers et similaires.

6. Dispositif (401) selon l'une quelconque des revendications précédentes,
**caractérisé en ce que**
le dispositif comprend au moins un silencieux (450) et ce silencieux comprend au moins un catalyseur, dans lequel le catalyseur (406) est combiné à un silencieux (450) et/ou intégré dans un silencieux (450).

7. Dispositif (401) selon l'une quelconque des revendications précédentes,
**caractérisé en ce que**
ledit au moins un filtre (405, 425) servant à la filtration stérile du gaz présente des tailles de pores inférieures à 50 µm, de préférence inférieures à 5 µm, de manière particulièrement préférée inférieures à 0,5 µm.

8. Dispositif (401) selon l'une quelconque des revendications précédentes,
**caractérisé en ce que**
un dispositif de mesure (407) est agencé dans au moins une des conduites de gaz (402, 402a, 402b) en aval d'au moins un filtre (405, 425) ou d'un catalyseur (406), lequel est adapté à mesurer la puissance de nettoyage du filtre (405, 425) ou du catalyseur (406) agencé en amont.

9. Dispositif (401) selon la revendication 8,
**caractérisé en ce que**
un dispositif d'arrêt (408) servant à fermer la conduite de gaz (402) est agencé dans au moins une des conduites de gaz (402, 402a, 402b) en aval d'au moins un dispositif de mesure (407).

10. Dispositif (401) selon la revendication 9,
**caractérisé en ce que**
le dispositif d'arrêt (408) servant à fermer la conduite de gaz (402, 402a, 402b) est relié à au moins un système de commande, lequel est adapté à fermer ou à ouvrir le dispositif d'arrêt (408) en fonction des valeurs déterminées par le dispositif de mesure (407).

11. Dispositif (401) selon au moins l'une des revendications précédentes,
**caractérisé en ce que**
le dispositif (401) comprend une salle blanche (6) à travers laquelle les récipients sont transportés.

12. Procédé de nettoyage de gaz dans des machines de soufflage pour la transformation d'ébauches de matière plastique en récipients en matière plastique,
dans lequel
le gaz étant guidé à travers au moins un système de traitement de gaz (405, 406, 416), lequel stérilise le gaz de préférence au moyen d'un filtre stérile (405) et/ou réduit des quantités résiduelles de désinfectant/stérilisant oxydatif dans le gaz au moyen d'un catalyseur (406), dans lequel
- au moins un système de traitement de gaz (416) étant un filtre (405) apte à effectuer un filtrage du gaz de manière stérile et ledit filtre (405) étant agencé dans une conduite de gaz (402, 402a) à travers laquelle un gaz comprimé peut être amené à une station de soufflage (8), **caractérisé en ce que**
- le filtre (405) utilisé comme filtre stérile central (405) est agencé dans le système de conduite (402), dans lequel ledit filtre stérile (405) est agencé dans une position en aval d'une alimentation d'un gaz de stérilisation (412), mais en amont d'un canal annulaire (454),
dans lequel les différents courants de gaz peuvent être régulés par différentes soupapes (455),
ou
- le système de traitement de gaz (416) comporte plusieurs filtres stériles (405), qui sont agencés en aval d'une alimentation d'un gaz de stérilisation (412) mais également en aval d'un canal annulaire (454) et en amont des stations de soufflage respectives, dans lequel des soupapes (455), régulant l'amenée de gaz aux différentes stations de soufflage, sont agencées en aval des filtres stériles (405).

13. Procédé selon la revendication 12,
**caractérisé en ce que**
le filtre stérile (405) agencé dans un système de conduite des machines de soufflage est nettoyé au moyen d'un désinfectant /stérilisant oxydatif, lequel est également utilisé pour désinfecter/stériliser des ébauches en matière plastique et/ou des récipients en matière plastique.
